# EUROPEAN PATENT APPLICATION

(11) **EP 3 721 835 A1**
(43) Date of publication of application: **14.10.2020**
(21) Application number: 19168856.3
(22) Date of filing: 12.04.2019
(51) Int. Cl.: A61F 2/24, A61B 5/00

(54) **HEART VALVE PROTHESIS AND METHOD FOR MEASURING FUNCTIONAL DEGRADATION OF THE PROSTHESIS**

(71) Applicant: BIOTRONIK AG, 8180 Bülach (CH)
(72) Inventor: Degen, Nicholas, 8222 Beringen (CH); Ulmer, Jens, 8703 Erlenbach (CH); Finnberg, Thomas, 8037 Zürich (CH); Stiehm, Michael, 18146 Rostock (DE); Erdbrügger, Wilhelm, 44379 Dortmund (DE)
(74) Representative: Keck, Hans-Georg

(57) **Abstract**

The present invention relates to a heart valve prosthesis (1) comprising a plurality of flexible valve leaflets (3), and a stent (2) for holding said valve leaflets (3). According to the present invention, the heart valve prosthesis (1) comprises at least one piezo element (5) and/or a member (7) with a bimetallic portion for generating at least one signal (U) indicative of operativeness of the heart valve prosthesis (1).

## Description

The present invention relates to an implantable heart valve prosthesis and a method for determining an operativenes of the heart valve prosthesis when the latter is implanted into a person.

Particularly, regarding a heart valve prosthesis it is desirable to be able to determine an operativeness of the implant of a functional degradation of the implant in an easy manner so that possible malfunctions can be detected in an early stage.

This problem is solved by a heart valve prosthesis according to claim 1 and a method according to claim 15.

Preferred embodiments of these aspects of the present invention are stated in the corresponding sub claims and are described below.

According to claim 1 an implantable heart valve prosthesis is disclosed, comprising:
- a plurality of flexible valve leaflets, and
- a stent for holding said valve leaflets.

According to the present invention, the heart valve prosthesis comprises at least a member with a bimetallic portion and/or one piezo element for generating at least one signal indicative of operativenes of the heart valve prosthesis.

The respective valve leaflets can move from a closed state for blocking blood flow through the prosthesis to an open state in which a blood flow can pass through an opening surrounded by the stent.

Particularly, the heart valve prosthesis is configured to be implanted into the heart of a patient/person via a catheter device, wherein particularly the heart valve prosthesis is configured to replace an aortic valve of a patient (e.g. TAVI). Here, the heart valve prosthesis comprises three valve leaflets that form three commissures, wherein each commissure can be connected to an associated commissural strut of the stent (see also below).

Particularly, according to an embodiment, the stent is a self-expandable stent or a stent that is configured to be expanded by means of a balloon. Particularly, the stent can be made from a suitable metal.

Further, according to an embodiment, the valve leaflets are formed out of a biological tissue.

Furthermore, according to an embodiment, the stent comprises struts that are connected to each other to form a plurality of cells of the stent.

Further, in an embodiment, each two neighboring valve leaflets form a commissure that is connected to an associated commissural strut of the stent. Particularly, a commissure is a location of junction of two neighboring (adjacent) valve leaflets.

Particularly, a piezo element is an element that generates an electrical voltage upon mechanical deformation of the piezo element due to the piezoelectric effect.

Further, according to an embodiment of the present invention, the at least one piezo element is connected to a commissural strut of the stent.

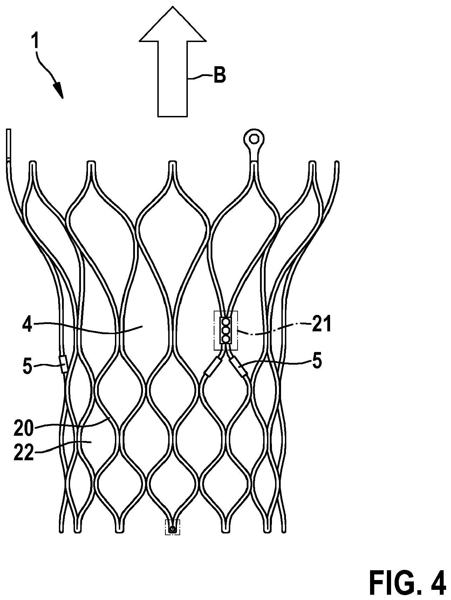

Further, according to an embodiment of the present invention, the at least one piezo element is connected to the commissural strut or to a strut connected to a commissural strut such that when a valve leaflet connected to said commissural strut moves from a closed state to an open state, the at least one piezo element is deformed (e.g. due to a load change on the commissural strut). An example of this embodiment is schematically shown in Fig. 4.

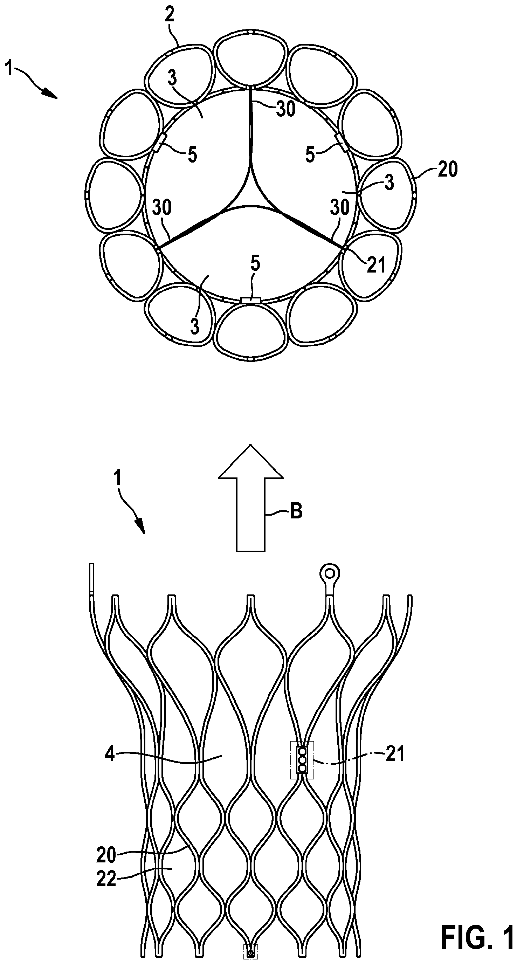

Further, according to an alternative embodiment of the present invention, the at least one piezo element is connected to the stent such that an associated valve leaflet of said plurality of valve leaflets contacts the piezo element in an open state of said associated valve leaflets. Particularly in this embodiment, said associated valve leaflet does not contact the at least one piezo element when residing in its closed state. An example of this embodiment is schematically shown in Fig. 1.

Further, according to an embodiment of the present invention, the member with a bimetallic portion is arranged in said opening and connected to the stent via a first end section of the member.

Further, according to an embodiment of the present invention, the member with the bimetallic portion comprises a body at a second end section of the member. The body can be of any shape. Preferably however, the body is cylindrical, or spherical. In the most preferred embodiment, the body is a sphere. The advantage of a spherical body is that the behavior of such a body is independent of the direction of blood flow relative to the body. The positioning of the spherical body in the blood flow therefore does not have to be as precise as for other shapes.

The body can be made from any material. Preferably the body is made of a metal. The body can also be bimetallic.

Further, according to an embodiment of the present invention, the member with a bimetallic portion is connected via the first end section to an (e.g. circumferential) section of the stent comprising the commissural struts.

Further, according to an embodiment of the present invention, the bimetallic portion of the member is configured to hold the body against, or close to, the stent periphery in its default setting. In other words, the body of the member rests against, or near to, the struts of the stent when the bimetallic portion of the member is not deformed by heating.

Further, according to an embodiment of the present invention, the bimetallic portion of the member is configured to bend the member (and therefore the body) inwards, towards the center of the stent, when it is heated so that the body moves into a blood flow flowing through said opening of the heart valve prosthesis when the heart valve prosthesis is implanted into the heart of a person. The bimetallic member can be heated by means of a coil attached to the heart valve prosthesis, which coil is configured to be excited by an external device that is not implanted into the patient and may be placed on the skin or in proximity of the patient/heart of the patient.

There are multiple advantages of having a member with a rest position in which the body is placed against the stent and a second position in which the body is located in the blood flow. One advantage is that in the rest position, the body does not cause resistance to the blood flow and therefore does not cause the blood flow to be weakened. In addition, when the body is in the blood flow, it causes platelet sheering, which can lead to thrombosis. A patient with a body that is always located in the blood flow may therefore have to take platelet inhibitors in order to counter the sheering effect. This platelet sheering does not take place, or only minimally takes place, when the body is removed from the blood flow between measurements.

Preferably the bimetallic portion is part of the first end section of the member that is connected to the stent. It is not necessary for the bimetallic portion of the member to extend along the entire length of the member. The bimetallic portion only needs to be sufficiently large so that the body at the other end of the member moves into the blood flow. Preferably, the bimetallic portion is configured to bend in such a way that the body at the other end of the member moves into the center of the blood flow (usually the radial center of the prosthesis). However, it is not absolutely essential that the body be located in the exact radial center of the prosthesis during measurement. The member should however be configured to allow to reproducibly place the body in the same position so that different measurements over time can be made reproducibly and can therefore be compared to each other.

The metals of the bimetallic portion of the member do not have to be pure metals. The two different metals, which can be alloys, have to have sufficiently different coefficients of thermal expansion in order for the body to move into the blood flow when the bimetallic portion of the member is heated.

The required size and shape of the bimetallic portion depends on the two metals with different coefficients of thermal expansion used to make the bimetallic portion. According to an embodiment, the bimetallic portion of the member extends over one third, preferably one half and most preferably the entire length of the bimetallic member.

Particularly, when the body (preferably the sphere) is arranged in said blood flow, vortices are generated when the flow separates from the body downstream of the body. These vortices are separated from the body with a characteristic frequency. Further, local pressure minima generated in the separating vortices result in a mechanical oscillation of the body which correlates with the flow velocity of the blood flow. The frequency of this oscillation can e.g. be measured using the Doppler method.

The measurement can be effectuated without requiring imaging. Only a frequency range between 30 and 400 Hz has to be measured. An oscillation with this frequency cannot be physiologically produced. The measurement of the blood flow with the member according to the invention is therefore simpler than known methods for which imaging is required.

A further advantage of the body being in the shape of a sphere is that the measurement can be made in any direction since when the body is a sphere, the angle at which the measurement is performed is irrelevant.

Alternatively, when the member is connected to a piezo element as described below, the oscillation of the body can be measured through measuring an electrical current on the skin.

Further, according to an embodiment of the present invention, the first end section of the member is connected to the stent via the at least one piezo element (or another member that is sensitive to changes in mechanical stress).

Further, according to an embodiment of the present invention, the heart valve prosthesis comprises a signal generating circuit connected to the at least one piezo element, which signal generating circuit is configured to generate said signal in the form of an electrical signal.

Further, according to an embodiment of the present invention, the signal generating circuit comprises (or is formed as) an oscillating circuit, wherein the piezo element is configured to transform a mechanical deformation of the piezo element (e.g. due to contact with the associated valve leaflet in said open state of said valve leaflet, or due to a vibration of the body/member with a bimetallic portion) into electrical energy for exciting said oscillating circuit to generate said electrical signal (e.g. an electrical current signal), wherein said oscillating circuit comprises two electrodes for contacting heart tissue and for applying said electrical signal to said tissue.

Further, according to an embodiment of the present invention, the heart valve prosthesis comprises a plurality of piezo elements for generating signals indicative of operativeness of the heart valve prosthesis, wherein each valve leaflet is associated to exactly one of the piezo elements.

Further, according to an embodiment of the present invention, the respective piezo elements is connected to the stent such that the associated valve leaflet contacts the respective piezo element in an open state of the associated valve leaflet. In order for the signal to be optimal, the piezo element should be located at the position where the end of the leaflet touches the stent in the open position.

Alternatively, or in addition, the respective piezo element is connected to an associated strut as described above, preferably to a commissural strut, such that when a valve leaflet connected to the respective commissural strut moves from a closed state to an open state (or vice versa) the respective piezo element is deformed (e.g. due to a load change on the commissural strut).

Further, according to an embodiment of the present invention, the heart valve prosthesis comprises a plurality of signal generating circuits, wherein each signal generating circuit is connected to exactly one of the piezo elements, wherein the respective signal generating circuit is configured to generate the respective signal (being indicative of operativeness of the heart valve prosthesis) in the form of an electrical signal.

Further, according to an embodiment of the present invention, the respective signal generating circuit comprises (or is formed as) an oscillating circuit, wherein the respective piezo element is configured to transform a mechanical deformation of the respective piezo element due to contact with the associated valve leaflet (in said open state of said valve leaflet) into electrical energy for exciting the respective oscillating circuit to generate the respective electrical signal (e.g. an electrical current signal), wherein the respective oscillating circuit comprises two electrodes for contacting heart tissue and for applying the respective electrical signal to said tissue.

Further, according to an embodiment of the present invention, each of said oscillating circuits comprises a different resonant frequency.

The potential/signal of the respective oscillating circuit applied to the body tissue via the respective two electrodes can be received e.g. by electrodes placed on the skin or in proximity of the patient.

The advantage of the present signal generating circuit lies within the fact that the generated signals do not stimulate the heart tissue and further are - concerning their frequency range - well above all other electrical muscle signals and can thus be detected with high sensitivity. Due to the fact that each signal generating circuit has its own unique resonant frequency (see above), the individual circuit / generated signal can be detected even in case circuits are excited at the same time.

According to yet another aspect of the present invention, a method for measuring a signal indicative of operativeness of a heart valve prosthesis implanted into a patient is disclosed, wherein implantation of the heart valve into the patient is explicitly not part of the method according to the present invention, and wherein the method preferably uses a heart valve prosthesis according to the present invention, wherein said at least one signal (or said plurality of signals) is measured and used to determine a measure for operativeness of the heart valve prosthesis.

Further features and advantages of the present invention shall be described below with reference to the Figures which show embodiments of the present invention, wherein:
- Fig. 1: shows an embodiment of a heart valve prosthesis according to the present invention comprising at least one piezo element for detecting movement of an associated valve leaflet;
- Fig. 2a: shows a signal generating current comprising an oscillating circuit connected to a piezo element;
- Fig. 2b: shows an example of a capacitor on a stent composed of conductive and isolating layers;

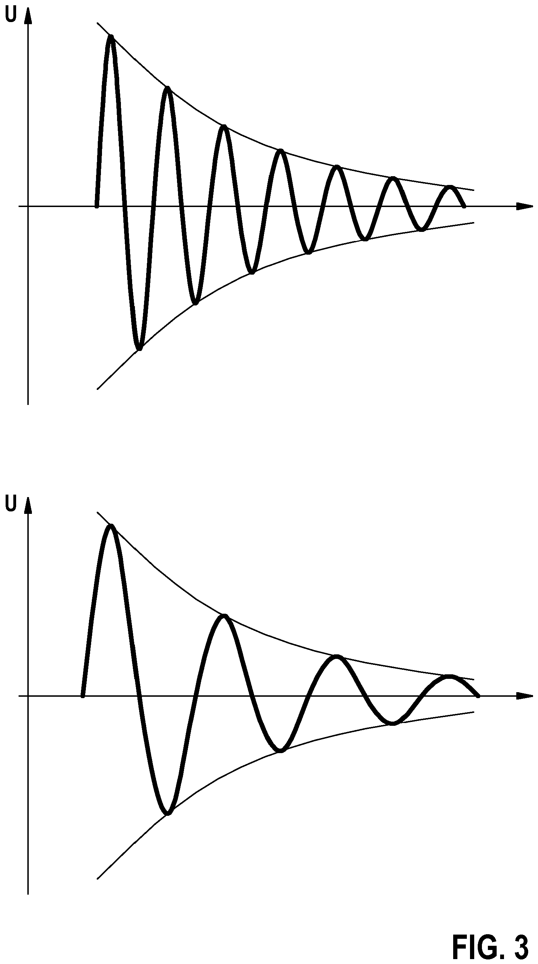

- Fig. 3: shows signals U generated by the signal generating current shown in Fig. 2a for different resonant frequencies of the oscillating circuit;
- Fig. 4: shows a further embodiment of a heart valve prosthesis according to the present invention, wherein deformation of the stent due to load changes upon movement of the valve leaflets is measured using at least one piezo element;
- Fig. 5: shows a further embodiment of a heart valve prosthesis according to the present invention using a member with a bimetallic portion and an oscillating body (in this case a sphere), wherein said oscillation can be measured using at least one piezo element and a signal generating circuit as shown in Fig. 2a or a Doppler method; and
- Fig. 6: shows the time-frequency diagram over one pulse cycle describing the oscillatory movement of a sphere located at a second end section of the member with a bimetallic portion as shown in Fig. 5.

Figs. 1, 4 and 5 show embodiments of heart valve prosthesis 1 according to the present invention, wherein the heart valve prosthesis 1 comprises a plurality of flexible valve leaflets 3, and a stent 2 for holding said valve leaflets 3. Particularly, the stent 2 comprises (e.g. metallic) struts 20 which enclose cells 22 of the stent 2. According to the present invention, the heart valve prosthesis 1 comprises at least one piezo element 5 and/or a member 7 with a bimetallic portion for generating at least one signal indicative of operativeness of the heart valve prosthesis 1.

Particularly the free play of the valve leaflets 3 is responsible for sealing properties of the prosthesis 1 as well as for the maximal flow rate of a blood flow B passing through the prosthesis 1 when the valve leaflets 3 are in an open state, which flow rate deteriorates in case the prosthesis suffers from functional degradation and loses operativeness.

According to the embodiment shown in Fig. 1, at least one piezo element 5, preferably a plurality of piezo elements 5, is used as contact switches that are contacted by the valve leaflets 3 when the latter move to the respective open state and hit the respective piezo element. In case of an aortic heart valve prosthesis 1, the prostheses may comprises three valve leaflets as shown in Fig. 1 and three associated piezo elements 5. The respective piezo element 5 can transform the mechanical energy due to the leaflets 3 hitting the respective piezo element 5 into electrical energy and thus functions as an energy collector. The energy transformed/collected in this way can be used to excite an oscillating circuit 6a of a signal generating circuit 6. Such a signal generating circuit 6 is particularly connected to each piezo element 5 shown in Fig. 1, wherein each oscillating circuit 6a has a different resonant frequency.

The generated signal/potential U of the respective oscillating circuit 6a can be applied to via two electrodes 61, 62, respectively, to body tissue of the patient and can be received by a suitable receiver (comprising e.g. electrodes arranged on the skin of the patient). The advantage of such a signal generating circuit 6 lies within the fact that the generated signals U do not stimulate the heart tissue and also lie (concerning frequency range) sufficiently above all other electrical muscle signal and can therefore be detected with sufficient sensitivity. Since each circuit 6a comprises its own resonant frequency, the individual piezo elements can be analyzed/detected also in case of simultaneous activation.

The load bearing structures for the valve leaflets 3 are the commissural struts 21. These structures 21 are bent due to load changes upon opening and closing of the valve leaflets 3. Assuming now that these load changes change when the mobility of the valve leaflets 3 changes, piezo elements 5 which are connected to the commissural struts 21 or to struts 20 leading to commissural struts 21 can detect a corresponding change in bending behavior.

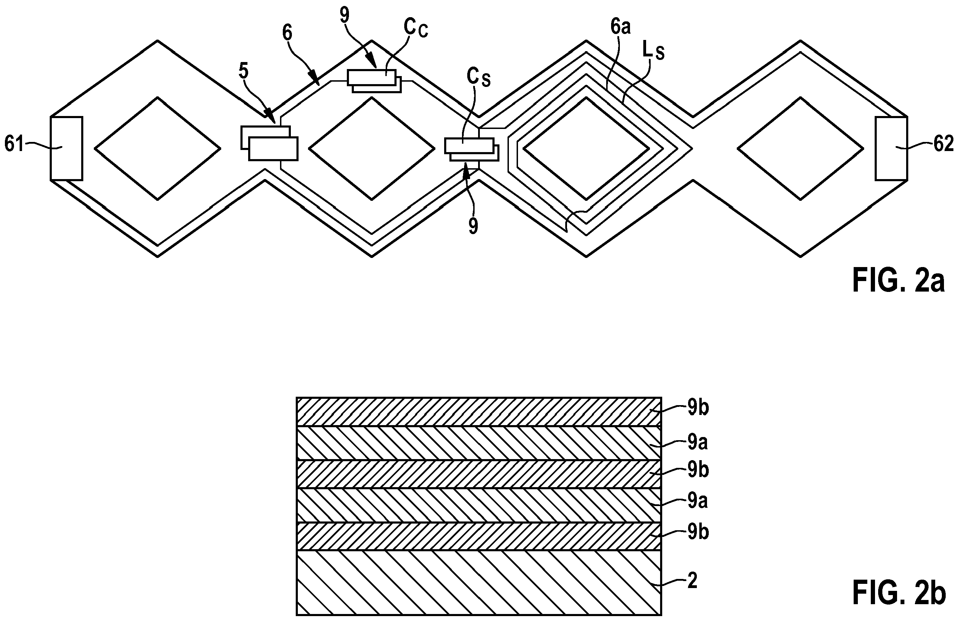

Also here, each piezo element 5 can be connected to an associated signal generating circuit 6 as shown in Fig. 2a. The electrodes 61, 62 are shown here as parts of conductor tracks at either end of the stent that are not isolated relative to the body. The capacitors 9 can be made up of alternating layers of conductive tracks 9a and isolation layers 9b that are deposited on the stent 2, as shown in Fig. 2B.

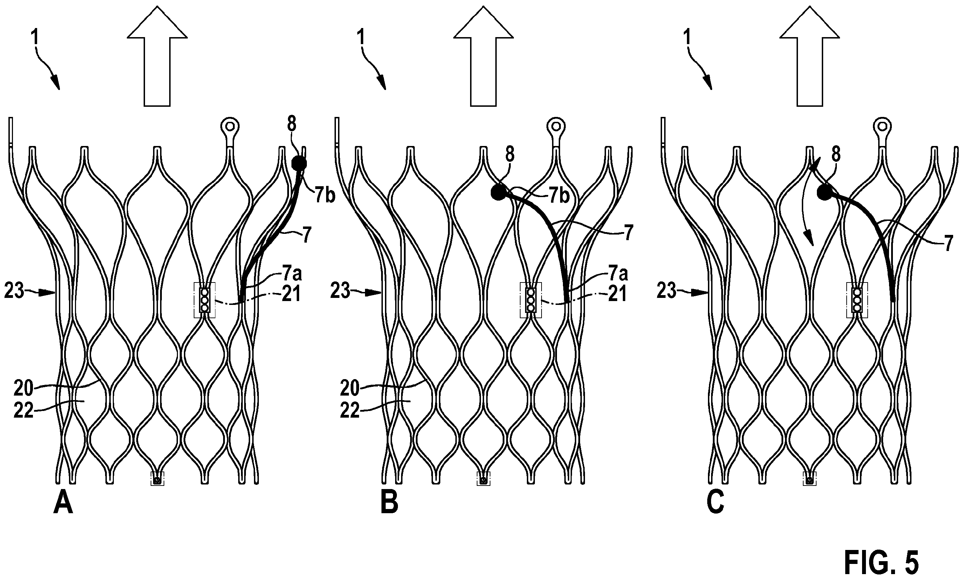

According to further embodiments, an oscillating member 7 is used as shown in Fig. 5. Here, a first end section 7a of the member 7 is connected to the stent 2 in the region 23 or height of the commissural struts 21 (with respect to an axial direction of the prosthesis 1). Further, the opposing second end section 7b is connected to a body 8 that preferably comprises the shape of a sphere or is a sphere. The bimetallic portion of the member 7 is designed particularly such that in an embodiment the metal comprising the larger thermal expansion is arranged on a side facing the vessel wall. Further, the prosthesis 1 comprises a coil that is configured to heat the bimetallic portion of member 7. The coil can receive electrical energy from outside the patient. Upon heating the bimetallic portion of member 7, the bimetallic property of this portion causes the member 7 and the body 8 to bend into the blood flow B as shown in Fig. 5B. When switching off an external field exciting the coil of the prosthesis 1 the bimetallic portion cools down again and the member 7 moves radially outwards, back into its initial position close to a side of the stent 2.

The coil configured to heat the bimetallic portion of the member 7 can rest on the bimetallic portion of member 7 itself. Alternatively, it can be attached to the stent 2 and connected to the bimetallic portion of member 7, for example through a wire. Preferably in this case, there are several coils attached to the perimeter of the stent 2. In such a case, the coils are arranged in series and distributed along the perimeter of the stent. Preferably, there are at least two coils, even more preferably between 4 and 20 coils arranged in series over the perimeter of the stent. The advantage of such an arrangement is that the likelihood that at least one of the coils will be in the required plan to be excited by the external electrical field is greatly increased. The direction of the applied field therefore does not have to be precisely controlled.

Particularly, in case the body 8 (preferably a sphere) is arranged in the blood flow B as shown in Fig. 5C, vortices are formed downstream the body 8 due to flow separation from the body geometry. These vortices separate from the body 8 with a characteristic frequency. Further, local pressure minima generated in the separating vortices cause the body 8 to oscillate in the blood flow B.

The frequency of this mechanical oscillation of the body 8 correlates with the flow velocity of the blood flow B and can therefore be measured easily, e.g. by using the Doppler method. Alternatively, the oscillation of the body 8 can also be measured using at least one piezo element 5 by coupling the member 7 to the at least one piezo element 5 connected to the stent 2. The oscillation frequency can then be transmitted as described above, for example using a signal generating circuit 6 as shown in Fig. 2a.

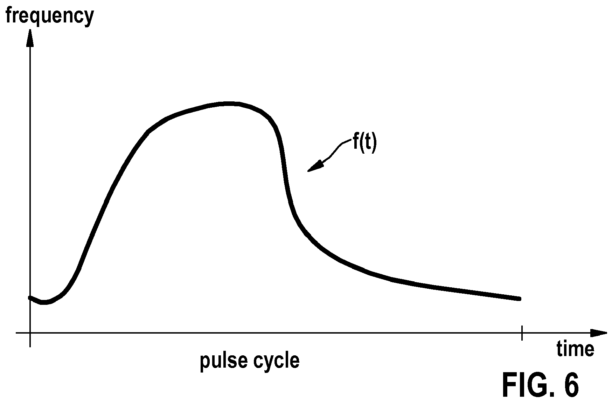

It is commonly known that the oscillatory vortex separation of a sphere in a flow field is induced at Reynolds numbers between Re = 130 and Re = 1000 and that the relation of frequency of the oscillatory vortex, the sphere diameter and the flow velocity can be described by the Strouhal number St, which is St = 0.20 for the used range of the Reynolds number. When the body (8) is a sphere, its diameter is preferably between 1.5 mm and 8 mm, preferably 2 mm to 7 mm, more preferably 3 mm to 6 mm, even more preferably 4 mm to 5 mm and most preferably 4.5 mm. It should however be noted that the smaller the sphere diameter, the further the sphere needs to be extended into the vessel lumen to induce oscillatory vortex structure. Assuming a flow velocity of 0,5 m/s and a kinematic viscosity for the blood of 4·10-6 m²/s, which are in the physiological range for the aortic flow, the obtained frequency of the oscillatory vortices is between 12,5 Hz and 67 Hz. Preferably therefore, the frequencies that should be measured in the method according to the invention lie within this range. Knowing that the frequency depends on the flow velocity, further analysis of the time-frequency diagram can be carried out, see Fig. 6.

In one embodiment according to the method of the invention, only the maximum oscillation frequency of the body 8 of member 7 is measured. The maximum oscillation frequencies at two or more measurement time points can then be compared. Since the body is reproducibly placed in the same position within the prosthesis, a decrease of the maximum oscillation frequency is indicative of a decreased blood flow B through the prosthesis. This may be indicative of a lower operativenes of the heart valve prosthesis.

It is also possible to measure other parameters of the oscillation of the body 8 on member 7 such as the acceleration and/or deceleration of the oscillation and/or the entire oscillation profile as depicted in Fig. 6. The oscillation profiles measured at two different time points can then be compared. Any changes in the oscillation profiles over time is indicative of a change in the operativenes of the heart valve implant.

## Claims

1. Heart valve prosthesis (1), comprising:
- a plurality of flexible valve leaflets (3),
- a stent (2) for holding said valve leaflets (3),
**characterized in that,**
the heart valve prosthesis (1) comprises
- a member (7) with a bimetallic portion; and/or
- at least one piezo element (5)
for generating at least one signal (U) indicative of operativeness of the heart valve prosthesis (1).

2. Heart valve prosthesis according to claim 1, **characterized in that** the stent (2) comprises struts (20, 21) that are connected to each other to form a plurality of cells (22) of the stent (2).

3. Heart valve prosthesis according to one of the preceding claims, **characterized in that** each two neighboring valve leaflets (3) form a commissure (30) that is connected to an associated commissural strut (21) of the stent (2).

4. Heart valve prosthesis according to claim 3, **characterized in that** the at least one piezo element (5) is connected to a commissural strut (21) or to a strut (20) leading to a commissural strut (21).

5. Heart valve prosthesis according to claim 4, **characterized in that** the at least one piezo element (5) is connected to the commissural strut (21) or to the strut (20) leading to a commissural strut (21) such that when a valve leaflet (3) connected to said commissural strut (21) moves from a closed state to an open state or vice versa, the at least one piezo element is deformed.

6. Heart valve prosthesis according to one of the claims 1 to 3, **characterized in that** the at least one piezo element (5) is connected to the stent (2) such that an associated valve leaflet (3) of said plurality of valve leaflets (3) contacts the at least one piezo element (5) in an open state of said associated valve leaflet (3).

7. Heart valve prosthesis according to one of the claims 1 to 3, **characterized in that** the stent (2) surrounds an opening (4) of the heart valve prosthesis (1) through which a blood flow (B) can be guided in an open state of the valve leaflets (3).

8. Heart valve prosthesis according to claim 7, **characterized in that** member (7) with a bimetallic portion is arranged in said opening (4) and connected to the stent (2) via a first end section (7a) of the member (7).

9. Heart valve prosthesis according to claim 8, **characterized in that** the member (7) comprises a body (8), preferably a sphere, at a second end section (7b) of the member (7) wherein the body (8) preferably rests against or close to the periphery of the stent (2) in the default state.

10. Heart valve prosthesis according to claim 3 and according to claim 8 or 9, **characterized in that** the member (7) is connected via the first end section (7a) to a section (23) of the stent (2) comprising the commissural struts (21).

11. Heart valve prosthesis according to claims 7 and according to claim 9 or 10, **characterized in that** the bimetallic portion of member (7) is configured to bend radially towards the middle of the stent when it is heated so that the body (8) is moved into a blood flow (B) flowing through said opening (4) of the heart valve prosthesis (1) when the heart valve prosthesis (1) is implanted into the heart of a person.

12. Heart valve prosthesis according to one of the claims 7 to 11, **characterized in that** the first end section (7a) of the member (7) is connected to the stent (2) via the at least one piezo element (5).

13. Heart valve prosthesis according to one of the preceding claims, **characterized in that** the heart valve prosthesis (1) comprises at least one piezo element (5) and a signal generating circuit (6) connected to the at least one piezo element (5), which signal generating circuit (6) is configured to generate said signal (U) in the form of an electrical signal.

14. Heart valve prosthesis according to claim 13, **characterized in that** the signal generating circuit (6) comprises an oscillating circuit (6a), wherein the at least one piezo element (5) is configured to transform a mechanical deformation of the at least one piezo element (5) into electrical energy for exciting said oscillating circuit (6a) to generate said signal (U), wherein said oscillating circuit (6a) comprises two electrodes (61, 62) for contacting heart tissue and for applying said signal (U) to said tissue.

15. Method for measuring a signal indicative of operativeness of a heart valve prosthesis (1) implanted into a patient, wherein the method measures at least one signal (U) generated by a heart valve prosthesis (1) according to one of the preceding claims, wherein said at least one signal (U) is used to determine a measure for operativeness of the heart valve prosthesis (1).
